# EUROPEAN PATENT SPECIFICATION

(11) **EP 0 378 146 B1**
(45) Date of publication and mention of the grant of the patent: **29.03.1995**
(21) Application number: 90100303.8
(22) Date of filing: 08.01.1990
(51) Int. Cl.: C07C 211/14

(54) **Polyamine derivatives as antineoplastic agents**
Polyamin-Derivate als antineoplastische Mittel
Dérivés de polyamine comme agents antinéoplastiques

(30) Priority: 10.01.1989 US 295721; 10.01.1989 US 295617
(43) Date of publication of application: 18.07.1990
(73) Proprietor: MERRELL PHARMACEUTICALS INC., Cincinnati Ohio 45215 (US)
(72) Inventor: Stemerick, David M., Fairfield, Ohio 45014 (US); Edwards, Michael L., Cincinnati, Ohio 45240 (US); Prakash, Nellikunja J., Cincinnati, Ohio 45241 (US); Bitonti, Alan J., Maineville, Ohio 45039 (US); McCann, Peter P., Cincinnati, Ohio 45241 (US); Sjoerdsma, Albert, Cincinnati, Ohio 45243 (US)
(74) Representative: Sgarbi, Renato

(56) References cited:
- EP-A- 0 270 349
- EP-A- 0 277 635
- EP-A- 0 311 068
- JOURNAL OF MEDICINAL CHEMISTRY, vol. 30, January 1987, pages 201-204; C.MELCHIORRE et al.: "Differential blockade of muscarinic receptor subtypes bypolymethylene tetraamines. Novel class of selective antagonists of cardiac M-2 muscarinic receptors"

## Description

### BACKGROUND OF THE INVENTION

Neoplastic disease states in humans are recognized throughout the world as being serious and sometimes life-threatening conditions. These neoplastic diseases, which are characterized by rapidly-proliferating cell growth, have been and continue to be the subject of worldwide research efforts directed toward the identification of therapeutic agents which are effective in the treatment of patients suffering therefrom. Effective therapeutic agents can be characterized as those which prolong the survivability of the patient, which inhibit the rapidly-proliferating cell growth associated with the neoplasm, or which effect a regression of the neoplasm. Research in this area is primarily focused toward identifying agents which would be therapeutically effective in humans. Typically, compounds are tested for antineoplastic activity in small mammals, such as mice, in experiments designed to be predictive of antineoplastic activity not only in those animals but also in humans against specific neoplastic disease states.

It is well known that naturally occurring polyamines, such as spermine and spermidine, play a role in cell growth and proliferation. These naturally occurring polyamines are found in animal cells and are produced in a biosynthetic pathway involving putrescine as a precursor.

Putrescine is formed by a decarboxylation of ornithine by ornithine decarboxylase (ODC).

EP-A 277635 discloses some polyamine derivatives which fall under formula I reported below. These compounds are described as possessing activity against infections caused by protozoa.

### SUMMARY OF THE INVENTION AND DETAILED DESCRIPTION

This invention relates to the use of certain polyamine derivatives for preparing a medicament for use in the treatment of patients suffering from certain neoplastic disease states. This invention also relates to pharmaceutical compositions containing these polyamine derivatives and a polyamine oxidase inhibitor.

More specifically, this invention relates to the use of compounds of formula (I)

RHN-Z-NH-(CH₂)ₘ-NH-Z-NHR (I)

wherein:
- m: is the integer 7,
- Z: is a saturated (C₂-C₆) alkylene moiety,
- each R: independently is methyl, ethyl, or n-propyl;
or a pharmaceutically acceptable acid addition salt thereof, for preparing a medicament for treating neoplastic diseases.

Particularly preferred are those compounds of formula I wherein m is the integer 7, Z is propylene, and each R group is ethyl, and the pharmaceutically acceptable acid addition salts thereof. A preferred compound of this invention is N,N'-bis[3-ethylaminopropyl]-1,7-heptanediamine. This invention thus relates to the use of the compounds of formula I for preparing a medicament for treating patients suffering from certain neoplastic disease states which comprises administering a therapeutically effective amount of a compound of the formula (I). Said treatment can optionally comprise conjunctive therapy with a polyamine oxidase inhibitor.

As indicated above, the center alkylene moiety (i.e., "(CH₂)ₘ") of compounds of the formula (I) is a saturated, straight-chain hydrocarbylene radical comprising 7 carbon atoms. As used herein, the term "Z" is understood to mean a saturated hydrocarbylene radical of straight or branched-chain configuration comprising 2 to 6 carbon atoms including, but not limited to, -CH₂CH₂-, -CH₂CH₂CH₂-, -CH₂(CH₂)₂CH₂- -CH₂(CH₂)₃CH₂-, -CH₂(CH₂)₄CH₂-, -CH(CH₃)CH₂-, -CH(CH₃)CH₂CH₂-, and the like.

The compounds of the formula I can be used according to the present invention as the pharmaceutically acceptable acid addition salts thereof. The term "pharmaceutically acceptable acid addition salt" encompasses both organic and inorganic acid addition salts including, for example, those prepared from acids such as hydrochloric, hydrofluoric, sulfuric, sulfonic, tartaric, fumaric, hydrobromic, glycolic, citric, maleic, phosphoric, succinic, acetic, nitric, benzoic, ascorbic, p-toluenesulfonic, benzenesulfonic, naphthalenesulfonic, propionic, and the like. The hydrochloric acid addition salts are preferred. The selection and preparation of pharmaceutically acceptable non-toxic acid addition salts are within the ability of one of ordinary skill in the art utilizing procedures and techniques well known and appreciated in the art.

In general, the compounds of formula (I) may be prepared by chemical reactions analogously known in the art, such as that described in European Patent Application Publication No. 277635. The choice of any specific route of preparation is dependent upon a variety of factors. For example, general availability and cost of the reactants, applicability of certain generalized reactions to specific compounds, and so forth, are all factors which are fully understood by those of ordinary skill in the art and all contribute to the choice of synthesis in the preparation of the compounds embraced by formula (I).

A preferred route for the synthesis of the compounds of the formula (I) is presented, in Reaction Scheme A. 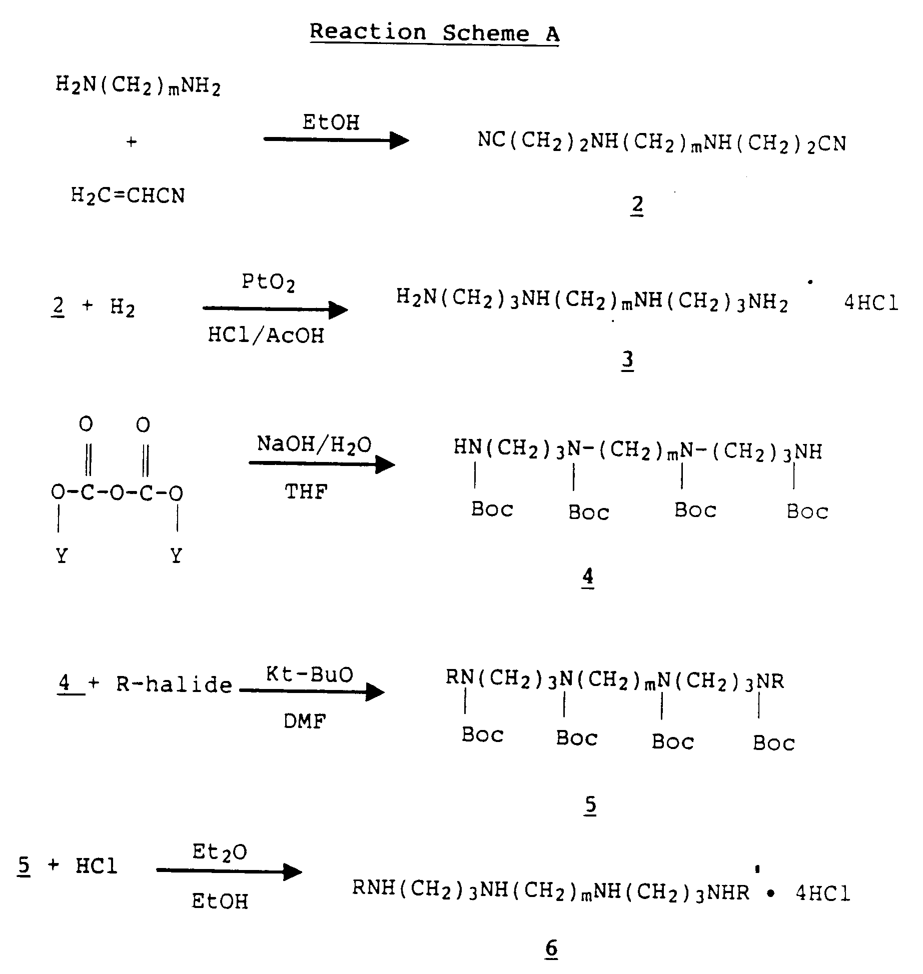
wherein m and R are as defined in formula (I), Boc is the t-butoxycarbonyl protecting group, and Y is tert-butyl.

The initial step of this process entails an N-alkylation of the appropriate diamine with 2 equivalents of acrylonitrile by heating reactants, either in a suitable solvent or neat, according to standard conditions well known in the art. The resulting cyano derivatives (2) are chemically reduced by reaction with hydrogen in the presence of a catalyst (PtO₂) in a suitable solvent, such as acetic acid containing 8 equivalents of hydrochloric or hydrobromic acid, to produce the corresponding hydrohalic salts according to standard procedures well known in the art. Of course, other reducing sytems, e.g., reduction with lithium aluminum hydride, may also be utilized to produce compounds of formula (3). Following the preparation of these compounds the hydrohalic salts are neutralized with base and the nitrogen atoms are protected, preferably with di-t-butyldicarbonate according to standard operating conditions well known in the art. The tetra N-protected amines (4) are alkylated by reacting (4) with the appropriate alkyl halides (chloro or bromo) in the presence of potassium butoxide according to standard alkylation procedures well known in the art. Since it is desired to make a compound of formula (1) wherein both R groups are the same, about 3 equivalents of the alkyl halide is reacted. Following alkylation the N-protective groups of compound (5) are removed by standard procedures, e.g., treatment with acid, preferably HCl, in the presence of a suitable solvent or solvent system, e.g., diethyloxide in ethanol, to obtain the desired products (6).

Alternatively, compounds of formula (3) and their otherwise prepared homologs may be subjected to a reductive alkylation using an appropriate aldehyde. The reduction is effected by hydrogenation in the presence of PtO₂ or sodium cyanoborohydride according to well known procedures. This procedure does not require protection of the nitrogen atoms of the intermediates.

A alternate route for the preparation of compounds of formula (I) is presented in Reaction Scheme B. 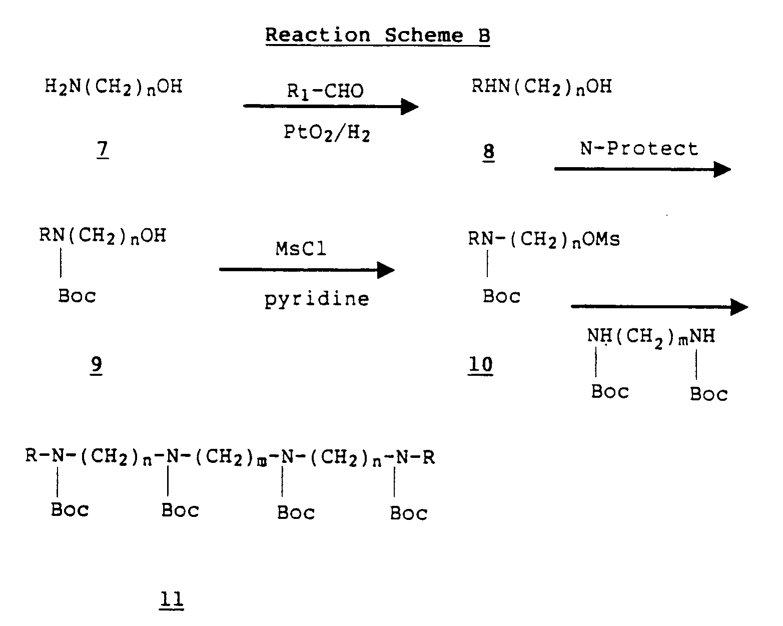
wherein m is as defined for formula (I), n is the integer 1,2 or 3 describing a straight chain alkylene moiety, Boc is the t-butoxycarbonyl protecting group, R is as defined in formula (I), Ms is mesyl and R₁ is methyl.

This synthesis is initiated by reductive alkylation techniques well known in the art using an amino alcohol (7) and an appropriate aldehyde to form R- substituted amino alcohols (8). The nitrogen atom is protected, preferably with di-t-butyldicarbonate, according to standard operating conditions well known in the art, to yield the N-protected amino alcohols (9) which are converted to their mesylates (10) by known reaction conditions, e.g., reaction with mesylchloride in the presence of pyridine, preferably in a solvent such as CH₂Cl₂.

The mesylate is subjected to alkylation with an N-protected diamine (i.e., BocNH(CH₂)ₘNHBoc) in the presence of potassium t-butoxide in a solvent such as DMF. The so-produced tetra N-protected tetramines (11) are deprotected as in Scheme A. In essence the foregoing reductive alkylation, N-protection, mesylation, alkylation and deprotection procedures all employ techniques and reaction conditions which are well known in the art.

Based upon standard laboratory experimental techniques and procedures well known and appreciated by those skilled in the art, as well as upon comparisons with compounds of known usefulness, a compound of formula (I) is particularly useful in the treatment of patients suffering from those neoplastic disease states which are dependent upon polyamine biosynthesis for their growth. Such neoplastic diseases include: leukemias, including but not limited to acute lymphoblastic, chronic lymphocytic, acute myloblastic and chronic mylocytic; carcinomas, including but not limited to those of the cervix, esophagus, stomach, small intestines, colon and lungs; sarcomas, including but not limited to oesteroma, osteosarcoma, lipoma, liposarcoma, hemangioma and hemangiosarcoma; melanomas, including amelanotic and melanotic; and mixed types of neoplasias such as, for example, carcinosarcoma, lymphoid tissue type, follicular reticulum, cell sarcoma and Hodgkins Disease.

As used herein, the term "patient" refers to a warm-blooded animal such as a mammal which is afflicted with a neoplastic disease state. It is understood that dogs, cats, rats, mice, horses, bovine cattle, sheep, and humans are examples of animals within the scope of the meaning of the term.

The term "neoplastic disease" as used herein refers to an abnormal state or condition characterized by rapidly proliferating cell growth or neoplasm such as a carcinoma, a sarcoma, a leukemia, and a melanoma.

Treatment of a patient afflicted with a neoplastic disease state comprises administering to such patient an amount of a compound of the formula (I) which is therapeutically effective in controlling the growth of the neoplasm or in prolonging the survivability of the patient beyond that expected in the absence of such treatment. As used herein, "controlling the growth" of the neoplasm refers to slowing, interrupting, arresting or stopping its growth and metastases and does not necessarily indicate a total elimination of the neoplasm. It is believed that prolonging the survivability of a patient, beyond being a significant advantageous effect in and of itself, also indicates that the growth of the neoplasm has been controlled.

In effecting treatment of a patient afflicted with a neoplastic disease state the compound of formula (I) can be administered parenterally in any manner which makes (I) bioavailable in effective amounts including for example, by intraperitoneal (i.p.), subcutaneous (s.c.), or intravenous (i.v.) injection. Administration by intravenous injection is preferred.

A therapeutically effective dose or amount can readily be determined by the attending diagnostician and is a function of a number of factors including, but not limited to, the species of mammal, its size, age and general health, the specific neoplasm involved, the degree of involvement, the stage of development of the neoplasm, the mode of administration, the bioavailability characteristics of the preparation administered, the dose regimen selected, and use of concomitant medication. The correct amount for any specific situation can be readily determined by those skilled in the art using conventional range finding techniques and analogous results observed under other circumstances. A therapeutically effective amount of (I) will vary from about 1 milligram per kilogram of body weight per day (mg/kg/day) to about 500 mg/kg/day and preferably will be about 5 mg/kg/day to about 50 mg/kg/day. It is believed that compounds of the formula (I) administered at the above doses to a patient suffering from a neoplastic disease are therapeutically effective in controlling the growth of one or more neoplastic disease states or in prolonging the survivability of the patient beyond that expected in the absence of such treatment.

A preferred embodiment of the present invention, relating to a method of treatment of patients suffering from a neoplastic disease state, comprises administering to said patient a therapeutically effective amount of a compound of the formula (I) in conjunctive therapy with an effective amount of a polyamine oxidase (PAO) inhibitor. The term "conjunctive therapy" contemplates coadministration of (I) along with a PAO inhibitor at essentially the same time, or treatment of the patient with a PAO inhibitor prior to or after treatment with (I). The PAO inhibitor is administered in an amount effective in substantially inhibiting PAO in the patient. When a compound of the formula (I) and a PAO inhibitor are administered in conjunctive therapy the PAO inhibitor may produce an additive or synergistic effect with (I). Thus, the dose of (I) required to produce a therapeutic effect in the patient may be less when administered in conjunctive therapy with an effective amount of a PAO inhibitor than that required when (I) is administered alone.

Various PAO inhibitors can be used including, but not limited to, N,N′-bis(2,3-butadienyl)-1,4-butanediamine, N-(2,3-butadienyl)-N′-(methyl)-1,4-butanediamine, or pharmaceutically acceptable acid addition salts thereof as described in U.S. Patent 4,551,550 which is incorporated herein by reference. N,N′-bis(2,3-butadienyl)-1,4-butanediamine is preferred as the PAO inhibitor for conjunctive therapy.

In effecting conjunctive therapy of a patient afflicted with a neoplastic disease state the PAO inhibitor can be administered parenterally in any manner which makes the PAO inhibitor bioavailable in effective amounts including, for example, by intraperitoneal (i.p.), subcutaneous (s.c.) or intravenous (i.v.) injection. Administration by intravenous injection is preferred.

An effective dose of the PAO inhibitor can readily be determined by the attending diagnostician and is a function of a number of factors including, but not limited to, the species of mammal, its size, age and general health, the specific neoplasm involved, the degree of involvement, the stage of development of the neoplasm, the mode of administration, the bioavailability character-istics of the compounds and preparation administered, the dose regimen selected, and use of concomitant medication. The correct amount of any specific situation can be readily determined by those skilled in the art using conventional range finding techniques and analogous results observed under other circumstances. An effective amount of a PAO inhibitor will vary from about 0.1 mg/kg/day to about 100 mg/kg/day and preferably will be about 1 mg/kg/day to about 10 mg/kg/day.

Pharmaceutical compositions for parenteral administration for the compounds of formula (I) comprise a therapeutically effective amount of one or more compounds of the formula (I) in an admixture with one or more pharmaceutically acceptable excipients, with or without, an effective amount of a PAO inhibitor. Such compositions are prepared in conventional manner well known in the art of pharmaceutical science. The amounts of the active ingredient(s) in a unit dosage form and the dosage regimen are adjusted to provide a sustained pharmacologic effect at the dose regimen selected.

Pharmaceutically acceptable excipients are substances that are chemically inert to the active compound(s) and have no detrimental side effects or toxicity to mammals under the conditions of use. Suitable excipients include solvents such as water, alcohol, and propylene glycol, surface active agents, suspending agents, lubricants, flavors, colorants, and the like. Such carriers and excipients are known to those in the art and are disclosed, for example, in texts such as Remington's Pharmaceutical Manufacturing, 13th Edition, Mack Publishing Co., Easton PA (1965).

Injectable dosage forms of a solution or suspension of (I) can be prepared, for example, in a physiologically acceptable diluent with a pharmaceutical carrier which can be a sterile liquid such as water and oils with or without the addition of a surfactants and other pharmaceutically acceptable adjuvants and with or without a PAO inhibitor. Illustrative of oils which can be employed in these preparations are those of petroleum, animal, vegetable or synthetic origin, for example, peanut oil, soybean oil and mineral oil. In general, water, saline, aqueous dextrose and related sugar solutions, ethanol and glycols such as propylene glycol or polyethylene glycol are preferred liquid carriers, particularly for injectable solutions.

As is well known in the art of pharmaceutical inventions wherein generic classes of compounds are involved, certain subgeneric and certain specific compounds are more efficient in their end-use applications than other members of the generic class. However, in this case the compound of formula (1) is unexpectedly superior in its end-use application as an antineoplastic agent than other closely related compounds such as N,N′-bis(3-aminopropyl)-1,8-octanediamine and N,N′-bis[3-(ethylamino)propyl]-1,8-octanediamine.

In order to illustrate the preparation and use of the compounds of formula (I), the following examples are provided. The examples are illustrative only and are not intended to limit the invention in any way. All temperatures are in degrees Celsius and the following abbreviations are used: (g) is grams, (mol) is moles, (ml) is milliliters, (l) is liters,

(TLC) is thin layer chromatography, (THF) is tetrahydrofuran, (DMF) is dimethylformamide, (mp) is melting point, (bp) is boiling point, percentages are by weight unless otherwise specified.

### EXAMPLE 1A

### N,N′-Bis[3-(ethylamino)propyl]-1,7-heptanediamine

### Steps A and B: 1,5,13,17-Tetraazaheptadecane tetrahydrochloride

Prepare the title compound by the method of Israel et al., J. Med. Chem. 7, 710 (1964).

### Step C: 1,5,13,17-Tetra(t-butoxycarbonyl)-1,5,13,17-tetraazaheptadecane

Combine 1,5,13,17-tetraazaheptadecane tetrahydrochloride (3.9 g , 0.01 mol) and sodium hydroxide (1.76 g , 0.44 mol) in water (44 ml) and stir until homogeneous. To this mixture add di-t-butyldicarbonate (9.6 g , 0.044 mol) in THF (88 ml) and stir for 3 hours. Dilute the mixture with ethyl acetate (EtOAc) [300 ml] and separate the organic layer. Dry the organic layer over anhydrous MgSO₄ and evaporate *in vacuo* to obtain a viscous oil. Purify the residue by flash chromatography (silica gel) eluting with 25% EtOAc/hexane to yield 3.0 g of the title compound. R_{f} is 0.20 on silica gel plates eluted with 25% EtOAc/hexane.

### Step D: 3,7,15,19-Tetra(t-butoxycarbonyl)-3,7,15,19-tetraazaheneicosane

Combine 1,5,13,17-tetra(t-butoxycarbonyl)-1,5,13,17-tetraazaheptadecane (3.0 g , 0.0046 mol) and sodium hydride (50% in oil) [0.45 g , 0.011 mol] in DMF (9 ml) and stir the mixture until hydrogen evolution ceases. Add ethyl iodide (0.9 ml, 0.011 mol) and stir the mixture for 18 hours. Evaporate the DMF *in vacuo* and partition the residue between ethyl acetate (600 ml) and water (200 ml). Separate the organic layer, dry the organic layer over anhydrous MgSO₄ and evaporate *in vacuo.* Purify the residue by flash chromatography (silica gel) eluting with 20% EtOAc/hexane to yield 1.68 g of the title compound. R_{f} is 0.5 on silica gel plates eluted with 25% EtOAc/hexane.

### Step E: N,N′-Bis[3-(ethylamino)propyl]-1,7-heptanediamine

Treat 3,7,15,19-tetra(t-butoxycarbonyl)-3,7,15,19-tetraazaheneicosane (1.68 g , 0.0024 mol) with HCl in methanol (50 ml, 1.0 N) and stir overnight. Filter the mixture and recrystallize the title compound from methanol/water (20:80, v/v) to yield 0.5 g of the title compound. R_{f} is 0.39 on silica gel plates eluted with 40% ammonia (concentrated) in methanol; mp 322-23°C with degradation.

### EXAMPLE 1B

### Antineoplastic Activity of N,N′-Bis[3-(ethylamino)propyl]-1,7-heptanediamine

Groups of 6 mice (BDF1, male) were inoculated with an intraperitoneal (i.p.) injection of L1210 leukemia cells (10⁵ cells) on day 0. Animals received one of the following treatment regimens on days 3 through 7:
Control Group - i.p. injection of inert vehicle every 3 hours for a total of 4 times per day [q 3h (x4) day 3-7];
Treatment Group A (test compound only)- i.p. injection of test compound at 5 or 10 mg/Kg body weight every 3 hours for a total of 4 times per day [5 or 10 mg/Kg q 3h (x4) day 3-7];
Treatment Group B (test compound + PAO inhibitor) - i.p. injection of test compound (5 or 10 mg/Kg body weight) and N,N′-bis(2,3-butadienyl)-1,4-butanediamine (5 mg/Kg body weight) every 3 hours for a total of 4 times per day [q 3h (x4) day 3-7].

The percent ratio of survival time for the treated group as compared to the control (T/C%) was calculated. The following results were obtained for N,N′-Bis[3-(ethylamino)propyl]-1,7-heptanediamine and N,N′-Bis[3-(ethylamino)propyl]-1,8-octanediamine as the test compounds:
N,N′-Bis[3-(ethylamino)propyl]-1,8-octanediamine: When administered at a dose of 6.25 mg/Kg i.p., q 3h (x4), on days 3, 4, 5, 6 and 7 to leukemic mice, N,N′-Bis[3-(ethylamino)propyl]-1,8-octanediamine prolonged the survival time by over 300% (T/C% > 300). However, animals treated with effective therapeutic doses of this compound displayed delayed toxicity symptoms such as continued body weight loss and wasting. When administered at a dose of 10 mg/Kg i.p., q 3h (x4), on days 3, 4 and 5, in combination with the polyamine oxidase inhibitor, N,N′-bis(2,3-butadienyl)-1,4-butanediamine (5 mg/Kg body weight), animals receiving treatment had prolonged survival times which were 287% (T/C% = 287) of the control group.
N,N′-Bis[3-(ethylamino)propyl]-1,7-heptanediamine: When administered at a dose of 5 mg/Kg i.p., q 3h (x4), on days 3 and 4 to leukemic mice, N,N′-Bis[3-(ethylamino)propyl]-1,7-heptanediamine prolonged the survival time by over 200% (T/C% > 200). The same dose on days 3, 4 and 5 results in apparent cure of the animals. Furthermore there were no signs of delayed toxicity. In combination with the polyamine oxidase inhibitor, N,N′-bis(2,3-butadienyl)-1,4-butanediamine (5 mg/Kg body weight), 100% cure rates were achieved.

This data shows that the compound of formula (1), i.e., N,N′-Bis[3-(ethylamino)propyl]-1,7-heptanediamine, has a much superior antineoplastic activity than the closely related compound N,N′-Bis[3-(ethylamino)propyl]-1,8-octanediamine.

## Claims

1. Use of a compound of the formula:
RHN-Z-NH-(CH₂)ₘ-NH-Z-NHR (I)
wherein:
m is the integer 7,
Z is a saturated (C₂-C₆) alkylene moiety,
each R group independently is methyl, ethyl, or n-propyl;
or a pharmaceutically acceptable acid addition salt thereof, for the preparation of a medicament for treating neoplastic diseases in patients in need thereof.

2. Use of a compound according to claim 1 wherein m in said compound is the integer 7, Z is propylene and each R is ethyl.

3. Use of a compound according to claim 1 which compound is N,N'-bis[3-(ethylamino)propyl]-1,7-heptanediamine.

4. Use according to claim 1 wherein the neoplastic disease is a carcinoma comprising carcinoma of the cervix, esophagus, stomach, small intestine, colon, or lungs; a sarcoma comprising oesteroma, osteosarcoma, lipoma, lyposarcoma, hemangioma, or hemangeosarcoma; a leukemia comprising lymphoblastic, chronic lymphocytic, acute myoblastic, or chronic mylocytic leukemias; or a melanoma comprising amelanotic or melanotic types.

5. A pharmaceutical composition comprising a compound of any of claims 1 to 3 and a polyamine oxidase inhibitor.

6. A product containing a compound of any of claims 1 to 3 and a polyamine oxidase inhibitor as a combined preparation for conjunctive antineoplastic therapy.

7. A pharmaceutical composition according to claim 5 or a product according to claim 6 wherein the polyamine oxidase inhibitor is N,N'-bis(2,3-butanedienyl)-1,4-butanediamine or N-(2,3-butadienyl)-N'-methyl-1,4-butanediamine.

## Patentansprüche

1. Verwendung einer Verbindung der Formel:
RHN-Z-NH-(CH₂)ₘ-NH-Z-NHR (I)
in der:
m eine ganze Zahl mit dem Wert 7 bedeutet,
Z eine gesättigte (C₂-C₆)-Alkyleneinheit und
jedes R unabhängig voneinander eine Methyl-, Ethyl- oder n-Propylgruppe darstellt, oder eines pharmazeutisch verträglichen Säureadditionssalzes davon zur Herstellung eines Arzneimittels zur Behandlung von neoplastischen Erkrankungen bei Patienten, die diese Behandlung benötigen.

2. Verwendung einer Verbindung nach Anspruch 1, wobei bei der Verbindung m eine ganze Zahl mit dem Wert 7 bedeutet, Z eine Propylengruppe und jedes R eine Ethylgruppe darstellt

3. Verwendung einer Verbindung nach Anspruch 1, wobei es sich bei der Verbindung um N,N'-bis[3-(Ethylamino)propyl]-1,7-heptandiamin handelt.

4. Verwendung nach Anspruch 1, wobei es sich bei der neoplastischen Erkrankung um ein Karzinom, umfassend die Karzinome der Gebärmutter, der Speiseröhre, des Magens, des Dünndarms, des Kolons oder der Lunge; ein Sarkom, umfassend Oesterome, Osteosarkome, Lipome, Liposarkome, Hämangiome oder Hämangiosarkome; eine Leukämie, umfassend lymphoblastische, chronisch lymphoblastische, akute myloblastische und chronisch mylocytische Leukämien; oder ein Melanom, umfassend amelanotische und melanotische Typen, handelt.

5. Arzneimittel, umfassend eine Verbindung nach einem der Ansprüche 1 bis 3 und einen Polyamin-Oxidase-Inhibitor.

6. Produkt, das eine Verbindung nach einem der Ansprüche 1 bis 3 und einen Polyamin-Oxidase-Inhibitor als eine kombinierte Zubereitung für eine gleichzeitige antineoplastische Therapie enthält.

7. Arzneimittel nach Anspruch 5 oder ein Produkt nach Anspruch 6, wobei es sich bei dem Polyamin-Oxidase-Inhibitor um N,N'-bis(2,3-Butadienyl)-1,4-butandiamin oder N-(2,3-Butadienyl)-N'-methyl-1,4-butandiamin handelt.

## Revendications

1. Utilisation d'un composé de formule :
RHN-Z-NH-(CH₂)ₘ-NH-Z-NHR (I)
dans laquelle
m est égal à 7,
Z est un reste alkylène saturé en C₂-C₆,
chaque groupe R est indépendamment un groupe méthyle, éthyle ou n-propyle ; ou d'un de ses sels d'addition d'acides acceptables en pharmacie, pour la préparation d'un médicament pour traiter les maladies néoplasiques chez des patients qui en ont besoin.

2. Utilisation d'un composé selon la revendication 1, dans laquelle m dans ledit composé est égal à 7, Z est un groupe propylène et chaque R est un groupe éthyle.

3. Utilisation d'un composé selon la revendication 1, qui est la N,N'-bis[3-(éthylamino)propyl]-1,7-heptanediamine.

4. Utilisation selon la revendication 1, dis laquelle la maladie néoplasique est un carcinome comprenant un carcinome du col, de l'oesophage, de l'estomac, de l'intestin grêle, du côlon ou des poumons ; un sarcome comprenant un ostéome, un ostéosarcome, un lipome, un liposarcome, un hémangiome ou un hémangiosarcome ; une leucémie comprenant les leucémies lymphoblastiques, lymphoïdes chroniques, myéloblastiques aiguës et myéloïdes chroniques ; ou un mélanome comprenant les types amélanotiques et mélanotiques.

5. Composition pharmaceutique comprenant un composé selon l'une quelconque des revendications 1 à 3 et un inhibiteur de polyamine-oxydase.

6. Produit contenant un composé selon l'une quelconque des revendications 1 à 3 et un inhibiteur de polyamineoxydase, comme préparation combinée pour une thérapie conjointe antinéoplasique.

7. Composition pharmaceutique selon la revendication 5 ou produit selon la revendication 6 dans lesquels l'inhibiteur de polyamine-oxydase est la N,N'-bis(2,3-butadiényl)-1,4-butanediamine ou la N-(2,3-butadiényl)-N'-méthyl-1,4-butanediamine.
